# EUROPÄISCHE PATENTSCHRIFT

(11) **EP 0 864 615 B1**
(45) Veröffentlichungstag und Bekanntmachung des Hinweises auf die Patenterteilung: **27.11.2002**
(21) Anmeldenummer: 98810109.3
(22) Anmeldetag: 12.02.1998
(51) Int. Cl.: C09B 67/22, C07C 229/16, C07C 233/43, C07C 271/28, C07C 275/40, C07C 311/05

(54) **Azofarbstoffmischungen**
Azodyestuff mixtures
Mélanges de colorants azoiques

(30) Priorität: 21.02.1997 CH 40997
(43) Veröffentlichungstag der Anmeldung: 16.09.1998
(73) Patentinhaber: Ciba Specialty Chemicals Holding Inc., 4057 Basel (CH)
(72) Erfinder: Herzig, Paul, 4057 Basel (CH); Clément, Antoine, 4058 Basel (CH); Dreier, Romeo, 4232 Fehren (CH); Arquint, Alfons, 4058 Basel (CH)

(56) Entgegenhaltungen:
- EP-A- 0 439 054
- EP-A- 0 555 179
- EP-A- 0 685 531
- US-A- 2 373 700

## Beschreibung

Die vorliegende Erfindung betrifft Mischungen von Azofarbstoffen, ihre Herstellung und ihre Verwendung zum Färben oder Bedrucken von halbsynthetischen oder synthetischen hydrophoben Fasermaterialien.

Azofarbstoffe und ihre Verwendung zum Färben von halbsynthetischen oder synthetischen hydrophoben Fasermaterialien sind bekannt. Es hat sich jedoch gezeigt, dass diese Farbstoffe den höchsten Ansprüchen, insbesondere im Bezug auf die Thermomigrierechtheit, nicht immer vollauf genügen. Es besteht daher Bedarf nach neuen Farbstoffen oder Farbstoffmischungen, welche sehr thermomigrierechte Färbungen oder Drucke ergeben und gute Auszug-, Aufbau- oder Waschechtheiteigenschaften zeigen.

Es wurde nun überraschenderweise gefunden, dass die erfindungsgemässen Mischungen die oben angegebenen Kriterien weitgehend erfüllen.

Gegenstand der vorliegenden Erfindung ist somit eine Farbstoffmischung enthaltend als Komponente (A) einen Farbstoff der Formel 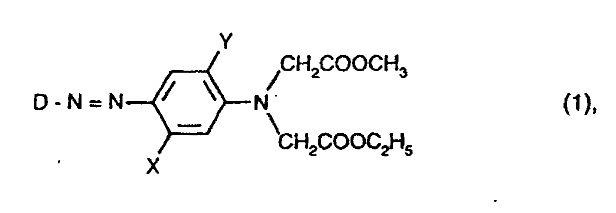 als Komponente (B) einen Farbstoff der Formel 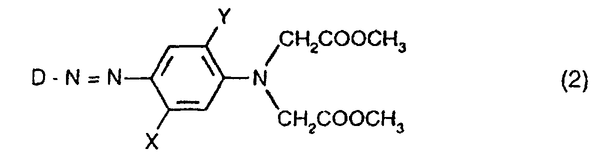 und als Komponente (C) einen Farbstoff der Formel 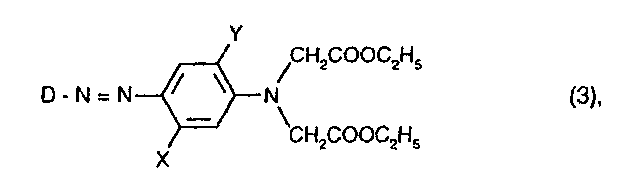 worin
D je den Rest einer Diazokomponente der Benzol-, Naphthalin-, Diphenyl-, Azobenzol-, Thiophen-, Benzthiazol-, Benzisothiazol-, Thiazol-, Thiadiazol-, Indazol-, Benztriazol-, Pyrazol-, Anthrachinon-, Naphtholsäureimid-, Chromon oder Diphenylenoxidreihe ist, X je Wasserstoff, Halogen, CF₃, R₃, OR₃, NH-CO-R₇, NH-CO-OR₈, NH-SO₂-R₇ oder NHCO-NR₄R₅, wobei R₃ C₁-C₆-Alkyl, R₄ und R₅ unabhängig voneinander je Wasserstoff, C₁-C₄-Alkyl oder C₁-C₄-Alkoxy-C₂-C₄-alkyl, R₇ Wasserstoff, C₁-C₆-Alkyl, C₁-C₄-Alkoxy-C₁-C₄-alkyl oder Phenyl und R₈ C₁-C₆-Alkyl oder C₁-C₄-Alkoxy-C₂-C₄-alkyl bedeuten, und Y je Wasserstoff, Halogen, Methoxy, Ethoxy oder O(CH₂)ₙ-OR₉ bedeutet, worin R₉ Wasserstoff, Methyl oder CH₂CH₂CN und n eine ganze Zahl von 1 bis 6 ist.

Unter Alkylresten sind erfindungsgemäss generell geradkettige, verzweigte oder cyclische Alkylgruppen zu verstehen.

R₃, R₇ und R₈ als C₁-C₆-Alkyl sind z.B. Methyl, Ethyl, Propyl, i-Propyl, n-Butyl, i-Butyl, sec.-Butyl, tert.-Butyl, Amyl, tert.-Amyl (1,1-Dimethylpropyl), 1,1,3,3-Tetramethylbutyl, Hexyl, 1-Methylpentyl, Neopentyl, Cyclopentyl, Cyclohexyl, sowie die dazugehörenden Isomeren.

R₄ und R₅ als C₁-C₄-Alkyl sind Methyl, Ethyl, Propyl, i-Propyl, n-Butyl, i-Butyl, sec.-Butyl, tert.-Butyl.

R₄, R₅, R₇ und R₈ als C₁-C₄-Alkoxy-C₂-C₄-Alkyl sind z.B. Methoxyethyl, Methoxypropyl, Methoxybutyl, Ethoxypropyl, Ethoxy-iso-propyl, Propoxyethyl, iso-Butoxypropyl oder n-Butoxyethyl.

X als Halogen ist Fluor, Brom, Jod oder vorzugsweise Chlor.

Y als Halogen ist Fluor, Brom, Jod oder vorzugsweise Chlor.

In bevorzugten Farbstoffmischungen stellt D einen Rest der Formel

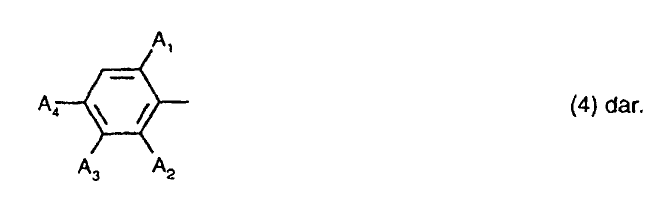

Darin bedeutet
A₁ Wasserstoff, Halogen, SO₂R₃, CF₃ oder CN,
A₂ Wasserstoff, Halogen, NO₂ oder CN,
A₃ Wasserstoff oder Halogen und
A₄ Wasserstoff, Halogen, Nitro, R₃, NHCOR₃ oder OR₃, worin R₃ die oben angegebene Bedeutung hat.

In besonders wertvollen Farbstoffmischungen bedeutet X Halogen, R₃ oder vorzugsweise den Rest NH-CO-R₇, worin R₃ und R₇ die oben angegebene Bedeutung haben und insbesondere voneinander unabhängig Methyl, Ethyl oder iso-Propyl sind.

In besonders wertvollen Farbstoffmischungen stellt D einen Rest der Formel (4) dar, worin A₁ Wasserstoff, Halogen oder CN, A₂ Wasserstoff, Halogen, CN oder Nitro, A₃ Wasserstoft und A₄ Nitro sind.

Ganz besonders wertvolle Farbstoffmischungen sind solche, die als D die Reste der Formel 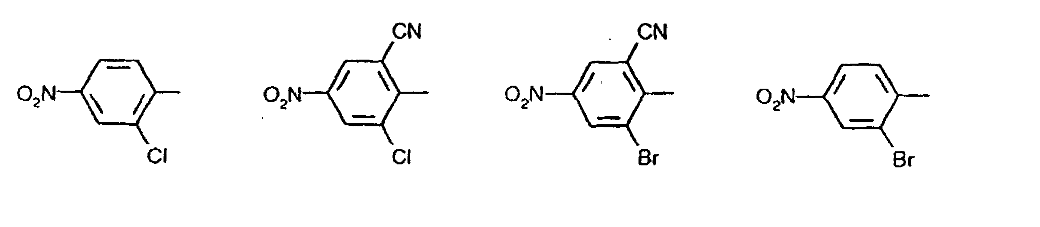 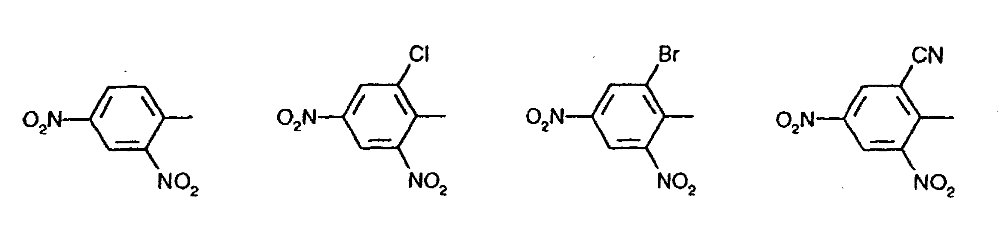 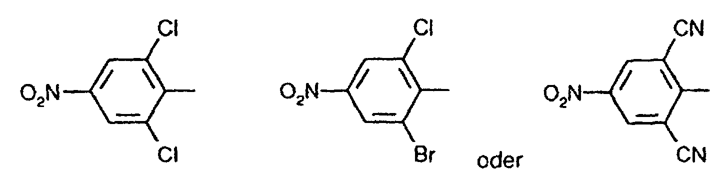 beinhalten.

Das Verhältnis der Komponenten (A) und (B), resp. der Komponenten (A) und (C) in den erfindungsgemässen Farbstoffmischungen kann in einem breiten Rahmen variieren z.B. von 99:1 bis zu 1:99, insbesondere von 95:5 bis zu 5:95, vor allem 90:10 bis zu 10:90.

Die Farbstoffkomponenten (B) und (C) der Formeln (2) und (3) sind z.B. aus der EP-A-0 555 179 bekannt. Die Farbstoffkomponente (A) der Formel (1) ist aus der US-A-2 373 700 bekannt.

Einen weiteren Gegenstand der vorliegenden Erfindung stellt das Verfahren zur Herstellung der erfindungsgemässen Farbstoffmischungen.

Diese werden beispielsweise so hergestellt, indem man eine Verbindung der Formel D-NH₂, insbesondere eine Verbindung der Formel 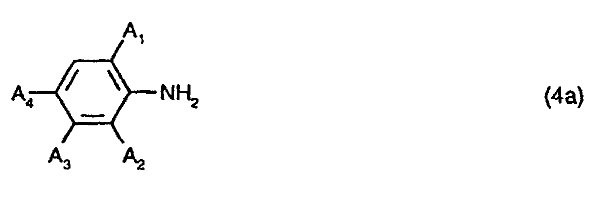 diazotiert und auf eine Kupplungskomponentenmischung, enthaltend die Kupplungskomponenten der Formeln 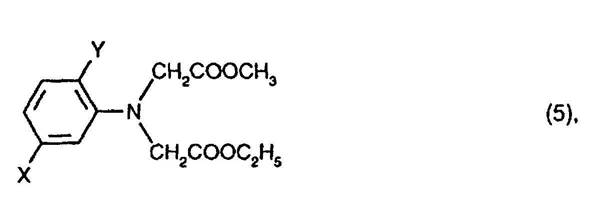 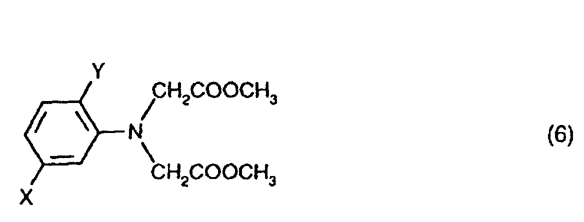 und 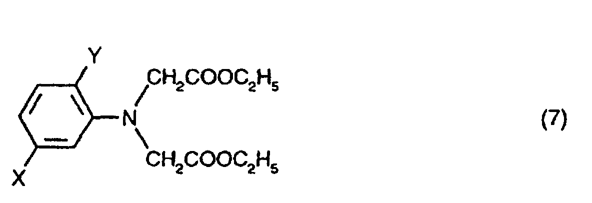 kuppelt, wobei D, A₁, A₂, A₃, A₄, X und Y die oben angegebenen Bedeutungen aufweisen.

Die Diazotierung der Verbindung der Formel (4a) erfolgt in an sich bekannter Weise, z.B. mit Natriumnitrit in saurem, z.B. salzsaurem oder schwefelsaurem, wässrigem Medium. Die Diazotierung kann aber auch mit anderen Diazotierungsmitteln, z.B. mit Nitrosylschwefelsäure ausgeführt werden. Bei der Diazotierung kann eine zusätzliche Säure im Reaktionsmedium anwesend sein, z.B. Phosphorsäure, Schwefelsäure, Essigsäure, Propionsäure, Salzsäure oder Mischungen dieser Säuren, z.B. Mischungen aus Propionsäure und Essigsäure. Zweckmässig wird die Diazotierung bei Temperaturen von -10 bis 30°C, z.B. von -10°C bis Raumtemperatur, durchgeführt.

Die Kupplung der diazotierten Verbindung der Formel (4a) auf die Mischung der Kupplungskomponenten der Formeln (5), (6) und (7) erfolgt ebenfalls in bekannter Weise, beispielsweise in saurem, wässrigem oder wässrig- organischem Medium, vorteilhaft bei Temperaturen von -10 bis 30°C, insbesondere unter 10°C. Als Säuren verwendet man z.B. Salzsäure, Essigsäure, Propionsäure, Schwefelsäure oder Phosphorsäure.

Die Diazokomponenten der Formel (4) sind bekannt und können auf an sich bekannte Art und Weise hergestellt werden.

Die Mischung der Kupplungskomponenten der Formeln (5), (6) und (7) ist neu und stellt ebenfalls einen Gegenstand der vorliegenden Erfindung dar.

Die Kupplungskomponenten der Formeln (5), (6) und (7) sind per se bekannt und können auf an sich bekannte Art und Weise hergestellt werden.

Einen weiteren Gegenstand der vorliegenden Erfindung stellt das Verfahren zur Herstellung der Kupplungskomponentenmischung enthaltend die Kupplungskomponenten der Formeln 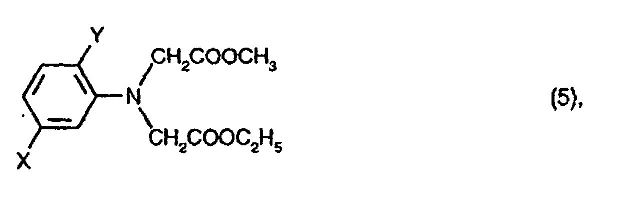 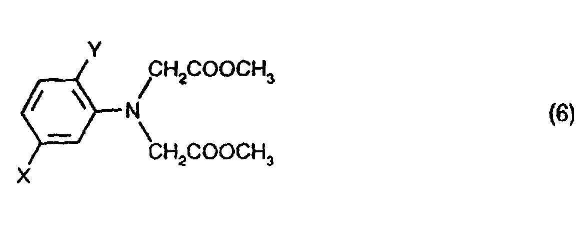 und 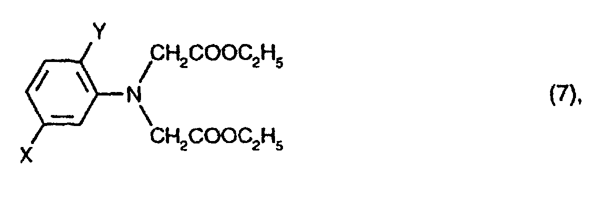 worin
X und Y die oben angegebene Bedeutungen und Bevorzugungen haben, dar, welches dadurch gekennzeichnet ist, dass man ein Anilin der Formel 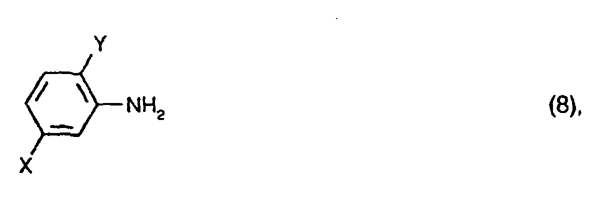 worin
X und Y die oben angegebene Bedeutung aufweist, mit einem Gemisch aus Chloressigsäuremethylester und Chloressigsäureethylester im Verhältnis 95:5 bis 5:95 umsetzt. Diese Umsetzung wird vorzugsweise bei erhöhter Temperatur, z. B. bei 60 bis 130°C, vorzugsweise bei 105 bis 125°C in Anwesenheit eines säurebindenden Mittels, wie z. B. Natriumacetat, Natriumbromid oder Soda, gegebenenfalls in einem inerten Lösungsmittel durchgeführt.

Die erfindungsgemässen Farbstoffmischungen können als Farbstoffe zum Färben und Bedrucken von halbsynthetischen und insbesondere synthetischen hydrophoben Fasermaterialien, vor allem Textilmaterialien, verwendet werden. Textilmaterialien aus Mischgeweben, die derartige halbsynthetische bzw. synthetische hydrophobe Textilmaterialien enthalten, können ebenfalls mit Hilfe der erfindungsgemässen Verbindungen gefärbt oder bedruckt werden.

Als halbsynthetische Textilmaterialien kommen vor allem Cellulose 2½-Acetat und Cellulosetriacetat in Frage.

Synthetische hydrophobe Textilmaterialien bestehen vor allem aus linearen, aromatischen Polyestern, beispielsweise solchen aus Terephthalsäure und Glykolen, besonders Ethylenglykol oder Kondensationsprodukten aus Terephthalsäure und 1,4-Bis-(hydroxymethyl)-cyclohexan; aus Polycarbonaten, z.B. solchen aus α,α-Dimethyl-4,4-dihydroxy-diphenylmethan und Phosgen, aus Fasem auf Polyvinylchlorid- sowie Polyamid-Basis.

Die Applikation der erfindungsgemässen Verbindungen auf die Textilmaterialien erfolgt nach bekannten Färbeverfahren. Beispielsweise färbt man Polyesterfasermaterialien im Ausziehverfahren aus wässriger Dispersion in Gegenwart von üblichen anionischen oder nicht-ionischen Dispergiermitteln und gegebenenfalls üblichen Quellmitteln (Carrier) bei Temperaturen zwischen 80 und 140°C. Cellulose-2½ -acetat färbt man vorzugsweise zwischen ungefähr 65 bis 85°C und Cellulosetriacetat bei Temperaturen bis zu 115°C.

Die erfindungsgemässen Farbstoffmischungen färben im Färbebad gleichzeitig anwesende Wolle und Baumwolle nicht oder nur wenig an (sehr gute Reserve), so dass sie auch gut zum Färben von Polyester/Wolle- und Polyester/Cellulosefaser-Mischgeweben verwendet werden können.

Die erfindungsgemässen Farbstoffmischungen eignen sich zum Färben nach dem Thermosol-Verfahren, im Ausziehverfahren und für Druckverfahren.

Das genannte Textilmaterial kann dabei in den verschiedenen Verarbeitungsformen vorliegen, wie z.B. als Faser, Faden oder Vlies, als Gewebe oder Gewirke.

Es ist vorteilhaft, die erfindungsgemässen Farbstoffmischungen vor ihrer Verwendung in ein Farbstoffpräparat zu überführen. Hierzu wird die Farbstoffmischung vermahlen, so dass ihre Teilchengrösse im Mittel zwischen 0,1 und 10 Mikron beträgt. Das Vermahlen kann in Gegenwart von Dispergiermitteln erfolgen. Beispielsweise wird die getrocknete Farbstoffmischung mit einem Dispergiermittel gemahlen oder in Pastenform mit einem Dispergiermittel geknetet und hierauf im Vakuum oder durch Zerstäuben getrocknet. Mit den so erhaltenen Präparaten kann man nach Zugabe von Wasser Druckpasten und Färbebäder herstellen.

Beim Bedrucken wird man die üblichen Verdickungsmittel verwenden, z.B. modifizierte oder nichtmodifizierte natürliche Produkte, beispielsweise Alginate, British-Gummi, Gummi arabicum, Kristallgummi, Johannisbrotkemmehl, Tragant, Carboxymethylcellulose, Hydroxyethylcellulose, Stärke oder synthetische Produkte, beispielsweise Polyacrylamide, Polyacrylsäure oder deren Copolymere oder Polyvinylalkohole.

Die erfindungsgemässen Farbstoffmischungen verleihen den genannten Materialien, vor allem dem Polyestermaterial, egale Farbtöne von sehr guten Gebrauchsechtheiten, wie vor allem guter Lichtechtheit, Thermofixier-, Plissier-, Chlor- und Nassechtheit wie Wasser-, Schweiss- und Waschechtheit; die Ausfärbungen sind femer gekennzeichnet durch sehr gute Reibechtheit. Besonders hervorzuheben ist die gute Thermomigrationsechtheit der erhaltenen Färbungen.

Die erfindungsgemässen Farbstoffmischungen können auch gut verwendet werden zur Herstellung von Mischnuancen zusammen mit anderen Farbstoffen.

Ausserdem eignen sich die erfindungsgemässen Farbstoffmischungen auch gut zum Färben von hydrophobem Textilmaterial aus überkritischem CO₂.

Die vorstehend genannte Verwendung der erfindungsgemässen Farbstoffmischungen stellt ebenso einen Gegenstand der vorliegenden Erfindung dar wie ein Verfahren zum Färben oder Bedrucken von halbsynthetischem oder synthetischem hydrophobem Fasermaterial, insbesondere Textilmaterial, das darin besteht, die erfindungsgemässe Farbstoffmischung auf das genannte Material aufzubringen oder sie in dieses einzuarbeiten. Das genannte hydrophobe Fasermaterial ist vorzugsweise textiles Polyestermaterial. Weitere Substrate, die durch das erfindungsgemässe Verfahren behandelt werden können, sowie bevorzugte Verfahrensbedingungen sind vorstehend bei der näheren Erläuterung der Verwendung der erfindungsgemässen Farbstoffmischungen zu finden.

Ein weiterer Gegenstand der Erfindung ist das durch das genannte Verfahren gefärbte bzw. bedruckte hydrophobe Fasermaterial, vorzugsweise Polyester-Textilmaterial.

Die erfindungsgemässen Farbstoffmischungen eignen sich ausserdem für moderne Aufzeichnungsverfahren, wie z.B. Thermotransfer-Printing.

Die nachfolgenden Beispiele dienen der Veranschaulichung der Erfindung. Darin sind, sofern nicht anders angegeben, die Teile Gewichtsteile und die Prozente Gewichtsprozente.

Die Temperaturen sind in Celsiusgraden angegeben. Die Beziehung zwischen Gewichtsteilen und Volumenteilen ist dieselbe wie zwischen Gramm und Kubikzentimeter.

### Beispiel 1:

50 Gewichtsteile 3-Aminoacetanilid werden zu einer in einem Reaktionsgefäss vorgelegten Mischung aus 98 Gewichtsteilen Chloressigsäure-methylester und 110 Gewichtsteilen Chloressigsäure-ethylester bei Raumtemperatur zugegeben. Anschliessend werden 58 Gewichtsteile wasserfreies Natriumcarbonat und 5,2 Gewichtsteile Natriumbromid zugegeben. Danach wird das Reaktionsgemisch innert 3 Stunden langsam auf 115°C aufgeheizt und bei dieser Temperatur 9 Stunden verrührt. Nach dieser Zeit wird die Reaktionsmischung auf 25°C abgekühlt und mit 320 Gewichtsteilen kaltem Wasser versetzt. Die entstandene Emulsion wird so lange gerührt, bis das vorher zugegebene Salz vollständig gelöst ist. Danach trennt man mittels eines Scheidetrichters die organische Phase ab und destilliert aus dieser das Gemisch aus Chloressigsäure-methylester und Chloressigsäureethylester unter Vakuum ab. Der Destillationsrückstand besteht aus einer Mischung von ca. 50% Methyl/Ethyl-ester der Formel 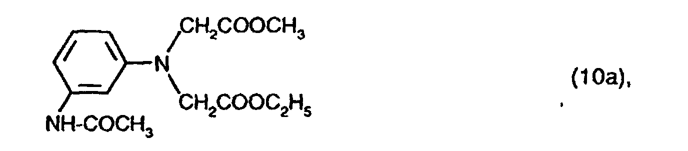 ca. 25% Dimethylester der Formel 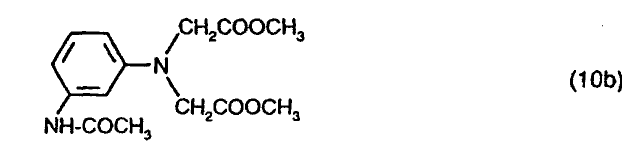 und ca. 25% Diethylester der Formel

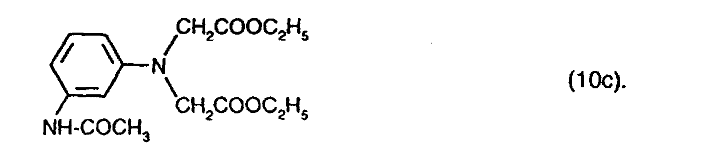

Verfährt man wie im Beispiel 1 beschrieben, verwendet aber anstelle einer equimolaren Mischung aus Chloressigsäure-methylester und Chloressigsäure-ethylester eine Mischung aus Chloressigsäure-methylester und Chloressigsäure-ethylester in dem in der Tabelle 1 angegebenen Verhältnis, so erhält man eine Mischung mit folgender Zusammensetzung der Ester der Formeln (10a) bis (10c):

**Tabelle 1:**

| Beispiel Nr. | Verhältnis (Mol %) Chloressigsäuremethylester : Chloressigsäureethylester | Zusammensetzung der Mischung der Verbindungen der Formeln (10a) : (10b) : (10c) |
|---|---|---|
| 2 | 75 : 25 | 37 : 56 : 7 |
| 3 | 80 : 20 | 35 : 60 : 5 |
| 4 | 85 : 15 | 26 : 71 : 3 |
| 5 | 90 : 10 | 22 : 77 : 1 |
| 6 | 25 : 75 | 37 : 7 : 56 |
| 7 | 20 : 80 | 35 : 5 : 60 |
| 8 | 15 : 85 | 26 : 3 : 71 |
| 9 | 10 : 90 | 22 : 1 : 77 |

Auf analoge Weise wie in Beispiel 1 beschrieben können anstelle 3-Aminoacetanilid auch folgende in der Tabelle 2 aufgeführten Amine verwendet werden:

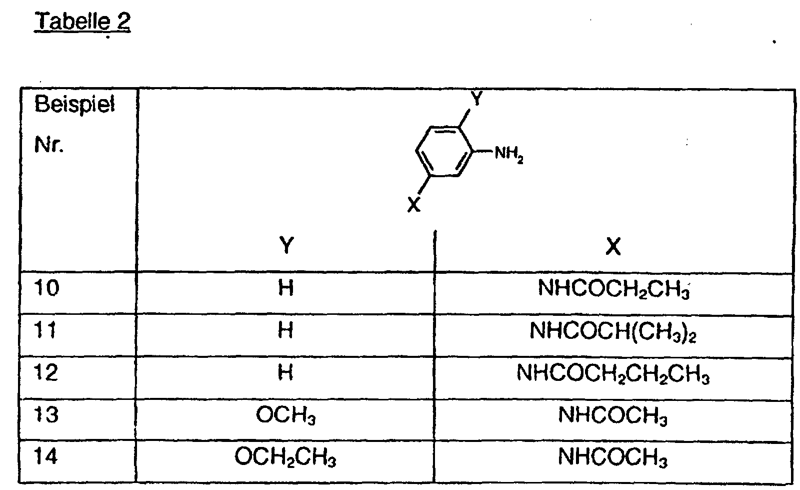

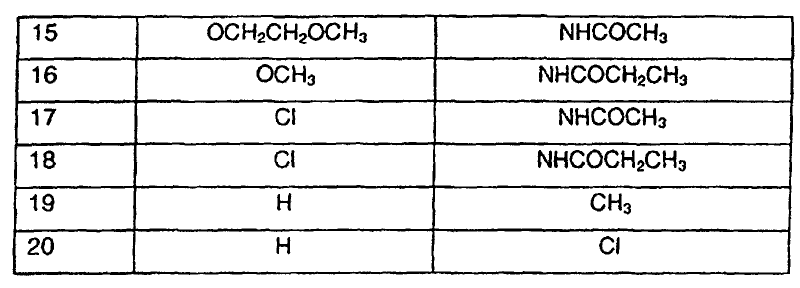

Auf analoge Weise wie in den Beispielen 2 bis 9 beschrieben können anstelle 3-Aminoacetanilid auch die in der Tabelle 2 aufgeführten Amine verwendet werden.

### Beispiel 21:

48,5 Gewichtsteile 2-Chlor-4-nitroanilin werden in 100 Gewichtsteilen Wasser angeschlämmt, mit 92 Gewichtsteilen 32%-iger HCI versetzt und 2 Stunden bei 20-30°C gerührt. Danach wird die Reaktionsmischung durch Zugabe von 125 Gewichtsteilen Eis auf 0°C abgekühlt. Anschliessend werden bei 0-5°C 81 Gewichtsteile einer 24,1%-igen G/G Natriumnitritlösung innert 30 Minuten zugetropft und 1 Stunde bei geringem Nitritüberschuss, der gegebenenfalls mit Sulfaminsäure korrigiert wird, verrührt. Die entstandene Diazolösung wird danach innerhalb 1,5 Stunden zu einer Lösung aus 88 Gewichtsteilen der Kupplungskomponente aus Beispiel 1, gelöst in 230 Gewichtsteilen wasserfreier Essigsäure zugetropft, wobei die Reaktionstemperatur mittels Zugabe von 400 Gewichtsteilen Eis bei 0-15°C gehalten wird. Nach beendeter Zugabe der Diazolösung wird das Reaktionsgemisch 1 Stunde nachgerührt. Der ausgefallene Farbstoff wird abgenutscht, mit Wasser gewaschen und getrocknet. Man erhält eine Scharlach-rote Farbstoffmischung mit folgender Zusammensetzung: 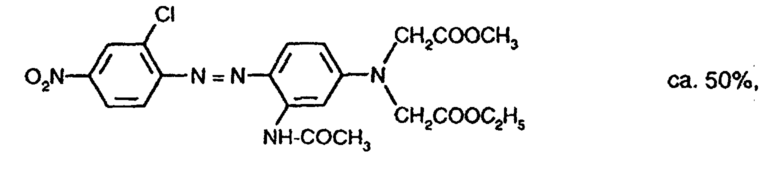 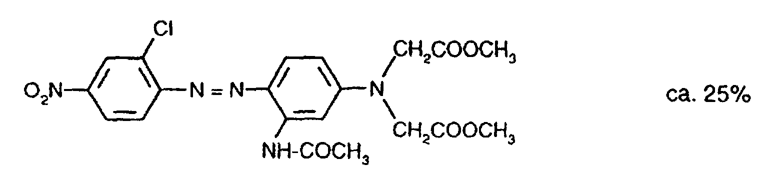 und 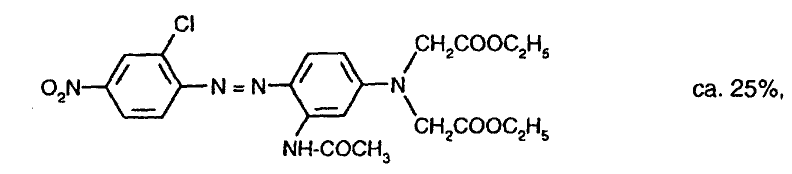 welche Polyester in einem brillanten scharlach Farbton mit guten Echtheiten, insbesondere Thermomigrierechtheit und Lichtechtheit färbt.

Auf analoge Weise wie im Beispiel 21 beschrieben werden folgende in der Tabelle 3 aufgeführten Mischungen hergestellt, welche ebenfalls Polyester in brillanten Farbtönen mit guten Echtheiten, insbesondere Thermomigrierechtheit und Lichtechtheit färben:

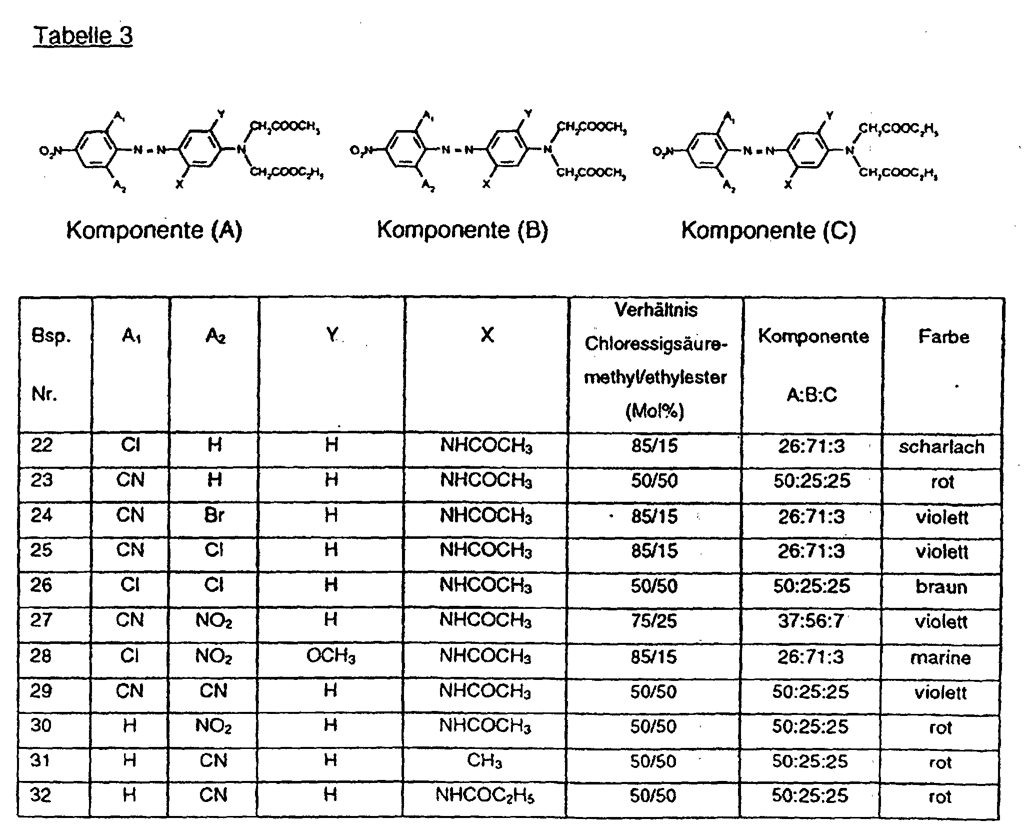

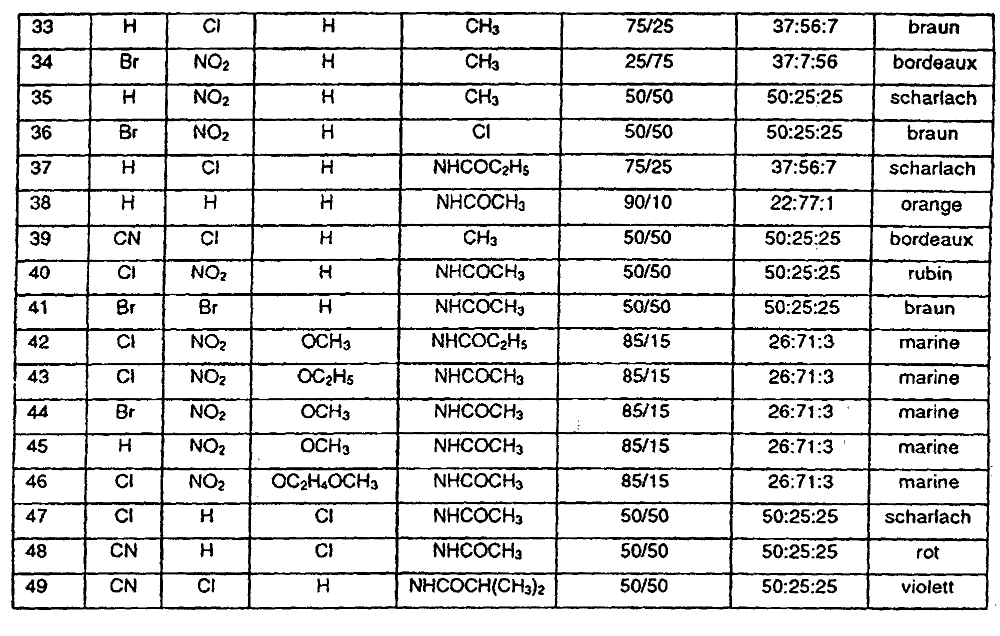

## Patentansprüche

1. Farbstoffmischung, enthaltend einen Farbstoff der Formel 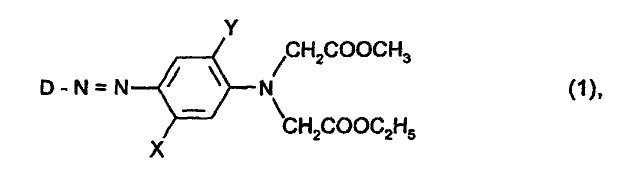 einen Farbstoff der Formel 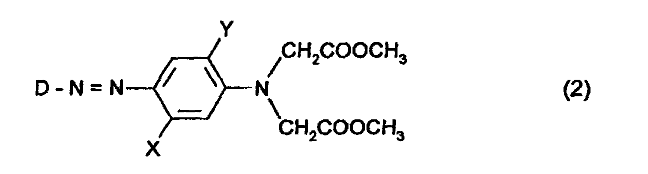 und
einen Farbstoff der Formel 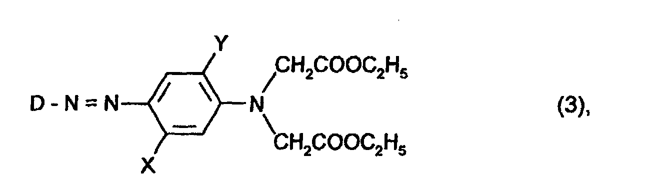 worin
D je den Rest einer Diazokomponente der Benzol-, Naphthalin-, Diphenyl-, Azobenzol-, Thiophen-, Benzthiazol-, Benzisothiazol-, Thiazol-, Thiadiazol-, Indazol-, Benztriazol-, Pyrazol-, Anthrachinon-, Naphtholsäureimid-, Chromon oder Diphenylenoxidreihe ist,
X je Wasserstoff, Halogen, CF₃, R₃, OR₃, NH-CO-R₇, NH-CO-OR₈, NH-SO₂-R₇ oder NHCO-NR₄R₅, wobei R₃ C₁-C₆-Alkyl, R₄ und R₅ unabhängig voneinander je Wasserstoff, C₁-C₄-Alkyl oder C₁-C₄-Alkoxy-C₂-C₄-alkyl, R₇ Wasserstoff, C₁-C₆-Alkyl, C₁-C₄-Alkoxy-C₁-C₄-alkyl oder Phenyl und R₈ C₁-C₆-Alkyl oder C₁-C₄-Alkoxy-C₂-C₄-alkyl bedeuten, und
Y je Wasserstoff, Halogen, Methoxy, Ethoxy oder O(CH₂)ₙ-OR₉ bedeutet, worin R₉ Wasserstoff, Methyl oder CH₂CH₂CN und n eine ganze Zahl von 1 bis 6 ist.

2. Farbstoffmischung gemäss Anspruch 1, worin D je einen Rest der Formel 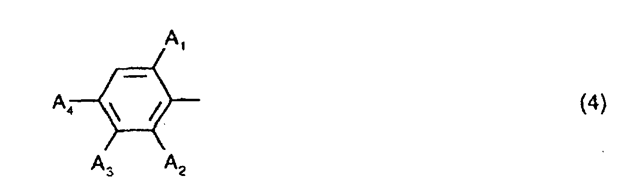 bedeutet, worin
A₁ Wasserstoff, Halogen, SO₂R₃, CF₃ oder CN, A₂ Wasserstoff, Halogen, NO₂ oder CN,
A₃ Wasserstoff oder Halogen ist, und A₄ Wasserstoff, Halogen, Nitro, R₃, NHCOR₃ oder OR₃ sind, wobei R₃ die in Anspruch 1 angegebene Bedeutung hat.

3. Farbstoffmischung gemäss einem der Ansprüche 1 und 2, worin X Halogen, R₃ oder ein Rest NH-CO-R₇ ist, wobei R₃ und R₇ die in Anspruch 1 angegebene Bedeutungen haben.

4. Farbstoffmischung gemäss Anspruch 2, worin A₁ Wasserstoff, Halogen oder CN, A₂ Wasserstoff, Halogen, CN oder Nitro, A₃ Wasserstoff und A₄ Nitro sind.

5. Farbstoffmischung gemäss Anspruch 2, worin D die Reste der Formel 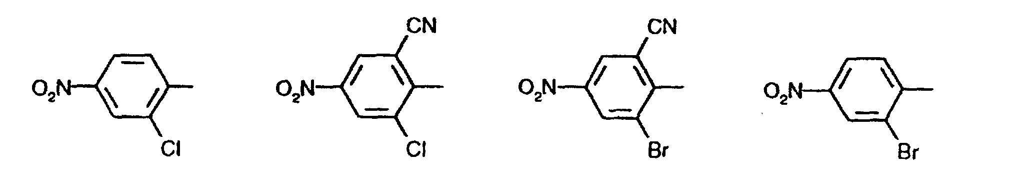 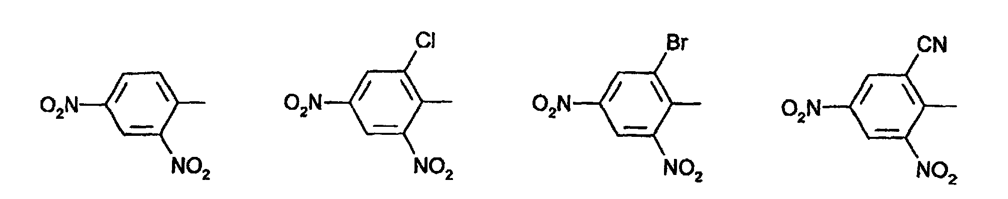 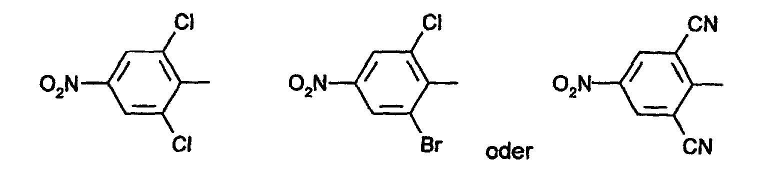 sind.

6. Verfahren zur Herstellung der Farbstoffmischung gemäss Anspruch 2, **dadurch gekennzeichnet, dass** man eine Verbindung der Formel 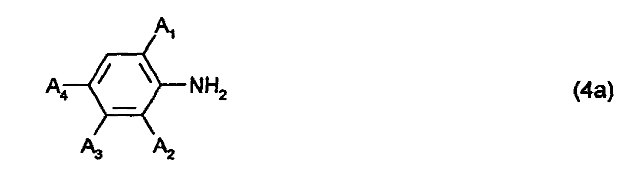 diazotiert und auf eine Kupplungskomponentenmischung, enthaltend die Kupplungskomponenten der Formeln 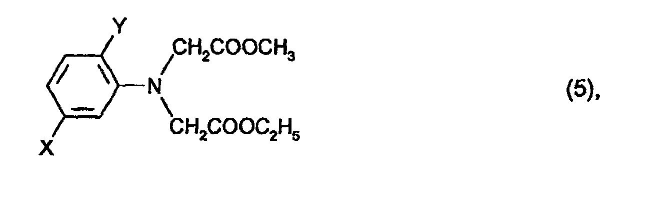 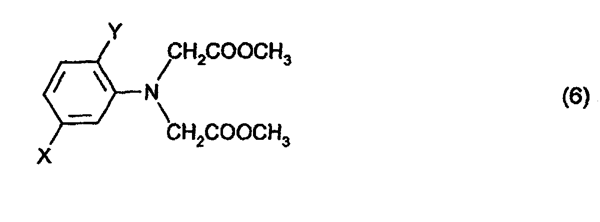 und 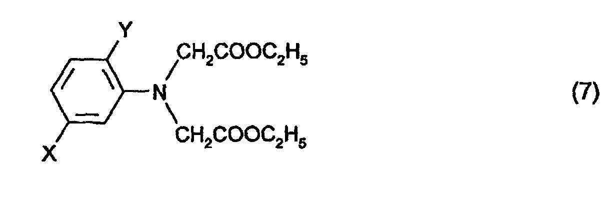 kuppelt, wobei A₁, A₂, A₃, A₄ die in Anspruch 2 und X und Y die in Anspruch 1 angegebenen Bedeutungen aufweisen.

7. Kupplungskomponentenmischung, enthaltend die Verbindungen der Formeln 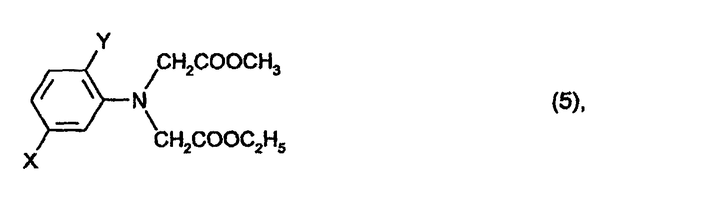 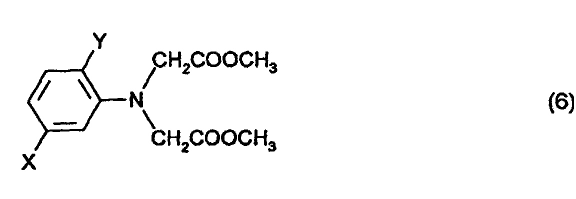 und 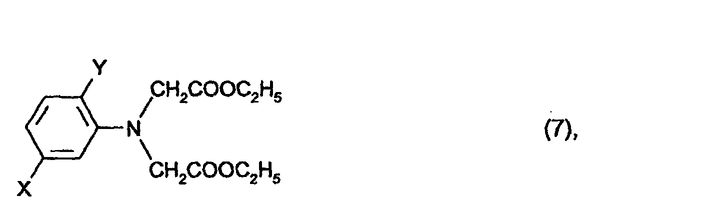 worin X je Wasserstoff, Halogen, CF₃, R₃, OR₃, NH-CO-R₇, NH-CO-OR₈, NH-SO₂-R₇ oder NHCO-NR₄R₅ ist, wobei R₃ C₁-C₆-Alkyl, R₄ und R₅ unabhängig voneinander je Wasserstoff, C₁-C₄-Alkyl oder C₁-C₄-Alkoxy-C₂-C₄-alkyl, R₇ Wasserstoff, C₁-C₆-Alkyl, C₁-C₄-Alkoxy-C₁-C₄-alkyl oder Phenyl und R₈ C₁-C₆-Alkyl oder C₁-C₄-Alkoxy-C₂-C₄-alkyl bedeuten, und Y je Wasserstoff, Halogen, Methoxy, Ethoxy oder O(CH₂)ₙ-OR₉ bedeutet, worin R₉ Wasserstoff, Methyl oder CH₂CH₂CN und n eine ganze Zahl von 1 bis 6 ist.

8. Verfahren zur Herstellung der Kupplungskomponentenmischung gemäss Anspruch 7, **dadurch gekennzeichnet, dass** man ein Anilin der Formel 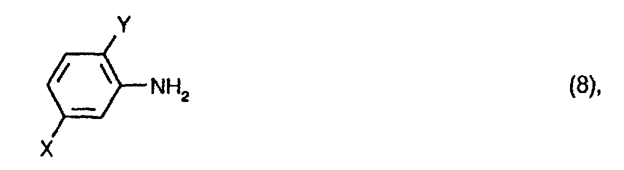 worin
X und Y die in Anspruch 7 angegebenen Bedeutungen aufweisen, mit einem Gemisch aus Chloressigsäuremethylester und Chloressigsäureethylester im Verhältnis 95:5 bis 5:95 umsetzt.

9. Verwendung der Farbstoffmischungen gemäss Anspruch 1 zum Färben oder Bedrucken von halbsynthetischem oder synthetischem hydrophobem Fasermaterial.

10. Verwendung nach Anspruch 9, wobei das halbsynthetische oder synthetische hydrophobe Fasermaterial ein Textilmaterial ist.

11. Verwendung nach Anspruch 10 zum Färben oder Bedrucken von Textilmaterial aus Polyesterfasern.

12. Verfahren zum Färben oder Bedrucken von halbsynthetischem oder synthetischem hydrophobem Fasermaterial, **dadurch gekennzeichnet, dass** man die Farbstoffmischung gemäss Anspruch 1 auf das genannte Material aufbringt oder diesem einverleibt.

13. Verfahren nach Anspruch 12, worin das hydrophobe Fasermaterial aus Polyesterfasern besteht.

14. Das gemäss Anspruch 12 oder 13 gefärbte oder bedruckte Material.

## Claims

1. A dye mixture which comprises
a dye of formula 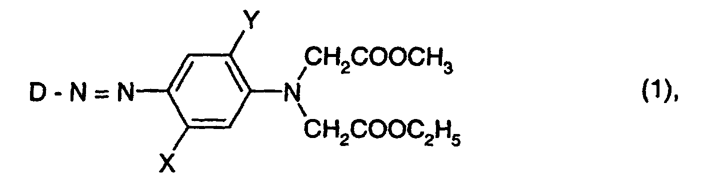 a dye of formula 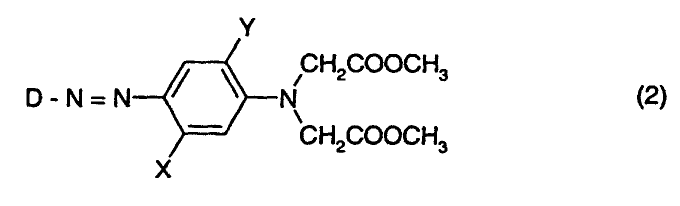 and
a dye of formula 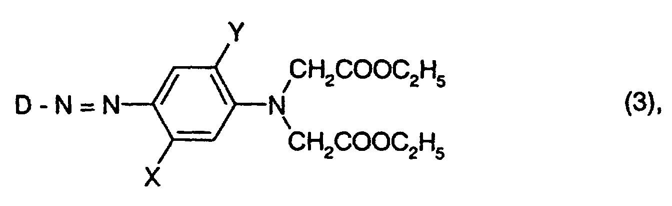 in which
D is in each case the radical of a diazo component of the benzene, naphthalene, diphenyl, azobenzene, thiophene, benzothiazole, benzisothiazole, thiazole, thiadiazole, indazole, benzotriazole, pyrazole, anthraquinone, hydroxynaphthoimide, chromone or diphenylene oxide series,
X is in each case hydrogen, halogen, CF₃, R₃, OR₃, NH-CO-R₇ NH-CO-OR₈, NH-SO₂-R₇ or NHCO-NR₄R₅ where R₃ is C₁-C₆alkyl, R₄ and R₅ are each independently of the other hydrogen, C₁-C₄alkyl or C₁-C₄alkoxy-C₂-C₄alkyl, R₇ is hydrogen, C₁-C₆alkyl, C₁-C₄alkoxy-C₁-C₄alkyl or phenyl and R₈ is C₁-C₆alkyl or C₁-C₄alkoxy-C₂-C₄alkyl, and
Y is in each case hydrogen, halogen, methoxy, ethoxy or O(CH₂)ₙ-OR₉, where R₉ is hydrogen, methyl or CH₂CH₂CN, and n is an integer from 1 to 6.

2. A dye mixture according to claim 1, wherein d is in each case a radical of formula 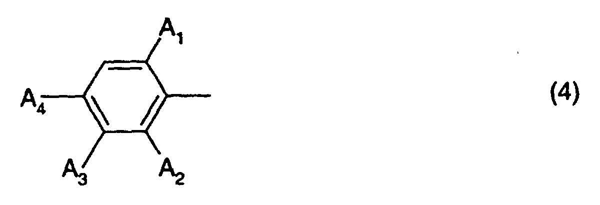 in which
A₁ is hydrogen, halogen, SO₂R₃, CF₃ or CN, A₂ is hydrogen, halogen, NO₂ or CN,
A₃ is hyrdogen or halogen, and A₄ is hydrogen, halogen, nitro, R₃, NHCOR₃ or OR₃, where R₃ has the meaning given in claim 1.

3. A dye mixture according to either claim 1 or claim 2, wherein X is halogen, R₃ or a radical NH-CO-R₇, where R₃ and R₇ have the meanings given in claim 1.

4. A dye mixture according to claim 2, wherein A₁ is hydrogen, halogen or CN, A₂ is hydrogen, halogen, CN or nitro, A₃ is hydrogen and A₄ is nitro.

5. A dye mixture according to claim 2, wherein D is the radicals of formula

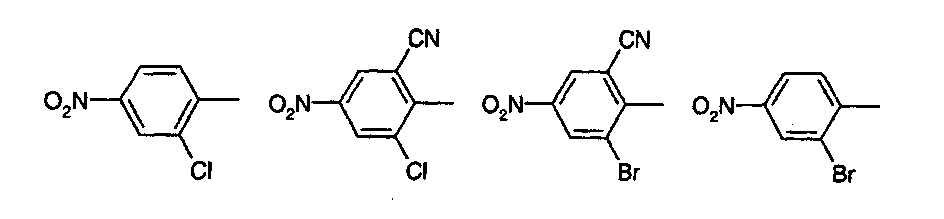

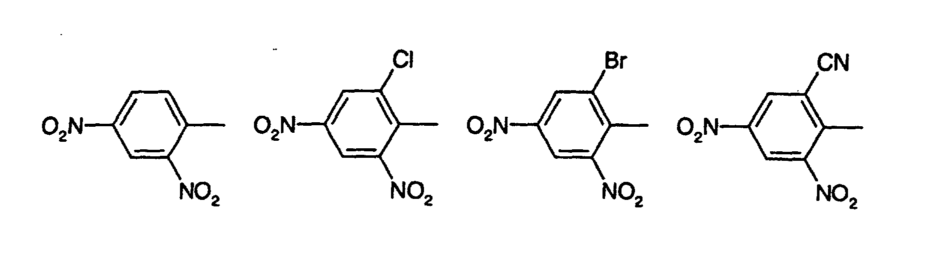

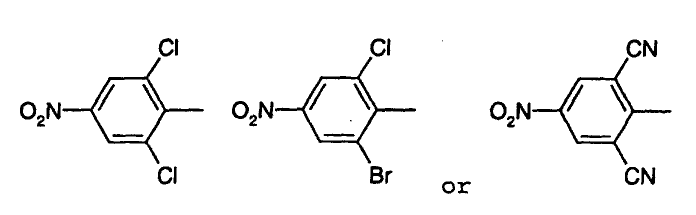

6. A process for the preparation of the dye mixture according to claim 2, which comprises diazotising a compound of formula 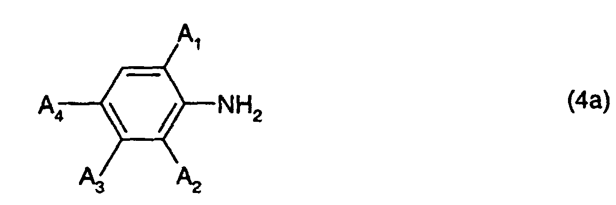 and coupling the diazonium compound so obtained to a coupling component mixture comprising the coupling components of formulae 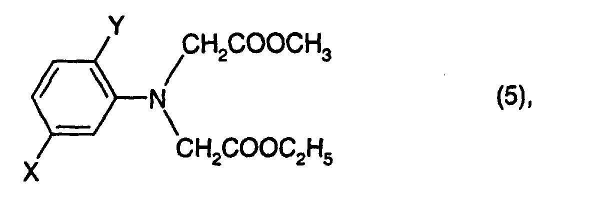 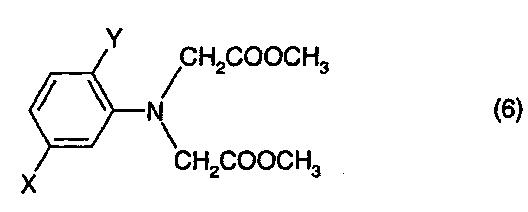 and 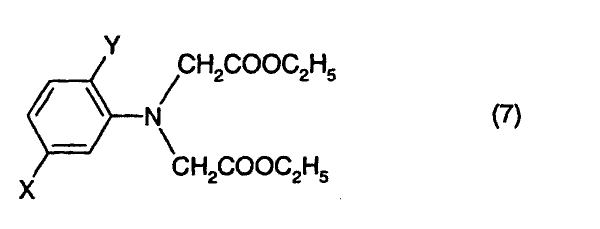 where A₁, A₂, A₃, A₄ have the meanings given in claim 2, and X and Y have the meanings given in claim 1.

7. A coupling component mixtue which comprises the compounds of formulae 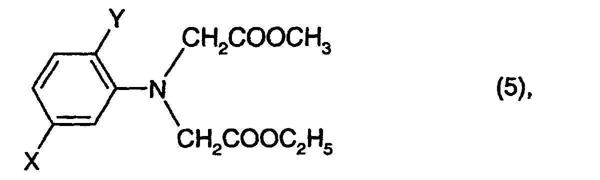 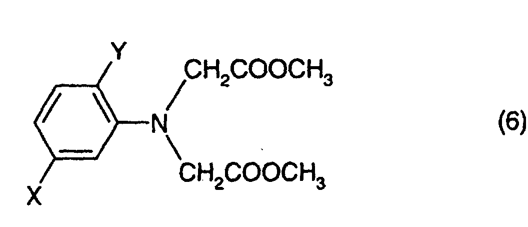 and 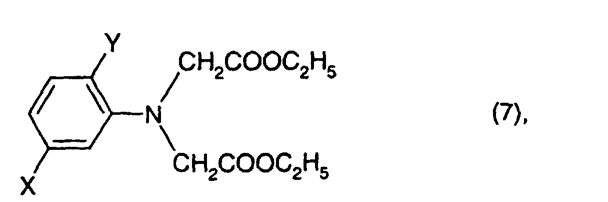 in which X is in each case hydrogen, halogen, CF₃, R₃, OR₃, NH-CO-R₇, NH-CO-OR₈, NH-SO₂-R₇ or NHCO-NR₄R₅, where R₃ is C₁-C₆alkyl, R₄ and R₅ are each independently of the other hyrogen, C₁-C₄alkyl or C₁-C₄alkoxy-C₂-C₄alkyl, R₇ is hydrogen, C₁-C₆alkyl, C₁-C₄alkoxy-C₁-C₄alkyl or phenyl, and R₈ is C₁-C₆alkyl or C₁-C₄alkoxy-C₂-C₄alkoxy-C₂-C₄alkyl and
Y is in each case hydrogen, halogen, methoxy, ethoxy or O(CH₂)ₙ-OR₉, where R₉ is hydrogen, methyl or CH₂CH₂CN, and n is an integer from 1 to 6.

8. A process for the preparation of the coupling component mixture according to claim 7, which comprises reacting an aniline of formula 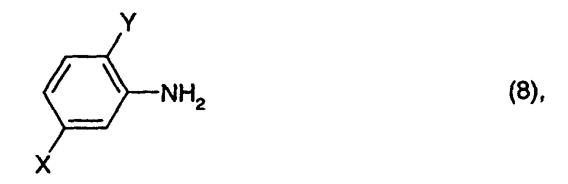 in which
X and Y have the meanings given in claim 7, with a mixure of methyl chloroacetate and ethyl chloroacetate in a ratio of 95:5 to 5:95.

9. Use of the dye mixture according to claim 1 for dyeing or printing semi-syntheti or synthetic hydrophobic fibre material.

10. Use according to claim 9, wherein the semi-synthetic or synthetic hydrophobic fibre material is a textile material.

11. Use according to claim 10 for dyeing or printing polyester fibre textile material.

12. A process for dyeing or printing semi-synthetic or synthetic hydrophobic fibre material, which comprises applying the dye mixture according to claim 1 to the stated material or incorporating it therein.

13. A process according to claim 12, wherein the hydophobic fibre material is composed of polyester fibres.

14. The material dyed or printed according to either claim 12 or claim 13.

## Revendications

1. Mélange de colorants, contenant un colorant de formule 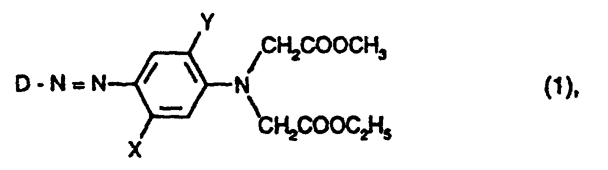 un colorant de formule 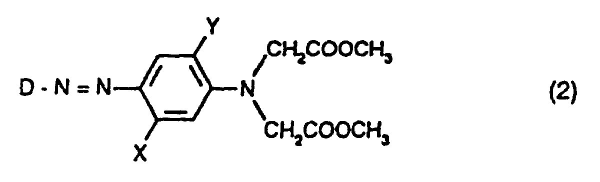 et
un colorant de formule 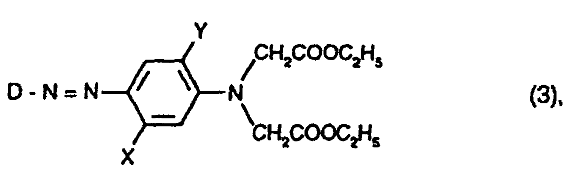 dans lequel
D est respectivement le reste d'un composant diazoïque du type benzène, naphtalène, diphényle, azobenzène, thiophène, benzothiazole, benzisothiazole, thiazole, thiadiazole, indazole, benzotriazole, pyrazole, anthraquinone, naphtolcarboximide, benzopyrone ou oxyde de diphénylène,
X représente respectivement un atome d'hydrogène, d'halogène, CF₃, R₃, OR₃, NH-CO-R₇, NH-CO-OR₈, NH-SO₂-R₇ ou NHCO-NR₄R₅, dans lesquels R₃ représente un groupe alkyle en C₁ à C₆, R₄ et R₅ représentent indépendamment l'un de l'autre respectivement un atome d'hydrogène, un groupe alkyle en C₁ à C₄ ou alcoxy en C₁ à C₄-alkyle en C₂ à C₄, R₇ représente un atome d'hydrogène, un groupe alkyle en C₁ à C₆, alcoxy en C₁ à C₄-alkyle en C₁ à C₄ ou phényle et R₈ représente un groupe alkyle en C₁ à C₆ ou alcoxy en C₁ à C₄-alkyle en C₂ à C₄, et
Y représente respectivement un atome d'hydrogène, d'halogène, un groupe méthoxy, éthoxy ou O(CH₂)ₙ-OR₉, dans lequel R₉ est un atome d'hydrogène, un groupe méthyle ou CH₂CH₂CN et n un nombre entier de 1 à 6.

2. Mélange de colorants selon la revendication 1, dans lequel D représente respectivement un groupe de formule 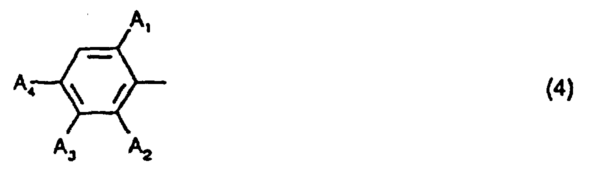 dans laquelle
A₁ représente un atome d'hydrogène, d'halogène, SO₂R₃, CF₃ ou CN,
A₂ représente un atome d'hydrogène, d'halogène, NO₂ ou CN,
A₃ est un atome d'hydrogène ou d'halogène, et
A₄ est un atome d'hydrogène, d'halogène, un groupe nitro, R₃, NHCOR₃ ou OR₃, dans lesquels R₃ a la signification indiquée à la revendication 1.

3. Mélange de colorants selon une des revendications 1 et 2, dans lequel X représente un atome d'halogène, R₃ ou un groupe NH-CO-R₇, dans lequel R₃ et R₇ ont les significations indiquées à la revendication 1.

4. Mélange de colorants selon la revendication 2,
dans lequel A₁ est un atome d'hydrogène, d'halogène ou CN, A₂ est un atome d'hydrogène, d'halogène, CN ou nitro, A₃ est un atome d'hydrogène et A₄ nitro.

5. Mélange de colorants selon la revendication 2,
dans lequel D est un groupe de formule

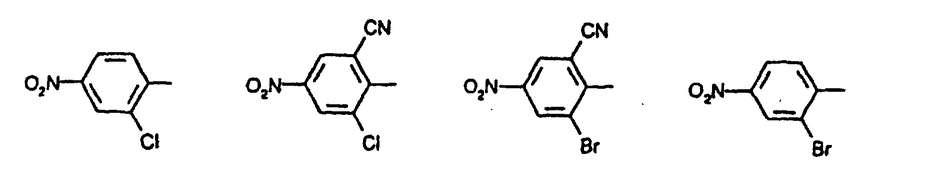

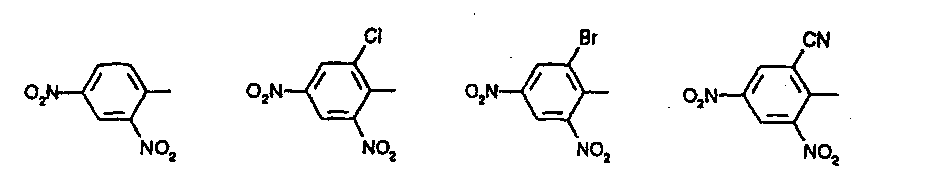

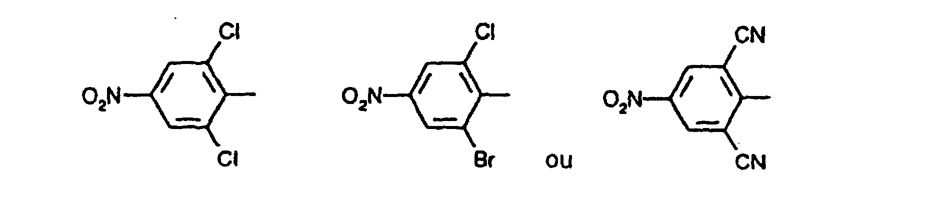

6. Procédé pour la préparation du mélange de colorants selon la revendication 2, **caractérisé en ce qu'**on réalise la diazotation d'un composé de formule 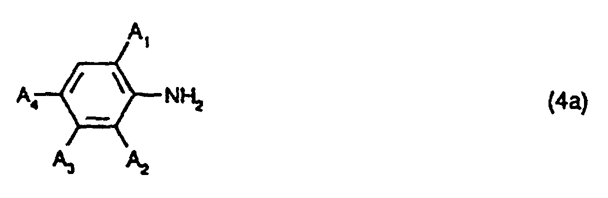 et la copulation sur un mélange de composants de copulation, contenant les composants de copulation des formules 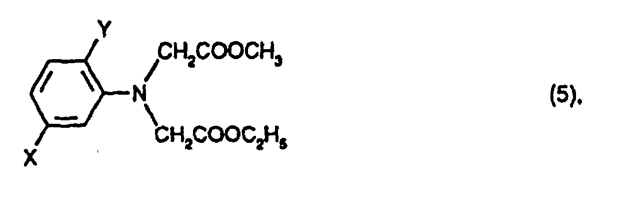 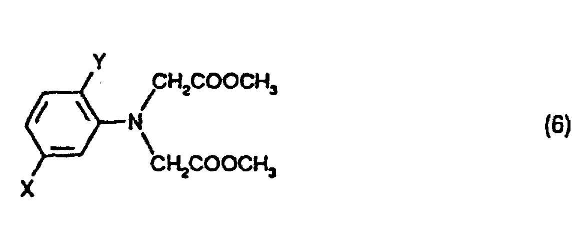 et 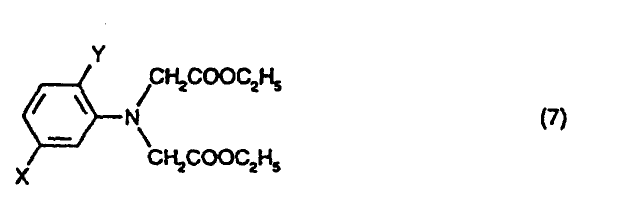 dans lesquelles A₁, A₂, A₃, A₄ présentent les significations indiquées à la revendication 2 et X et Y celles indiquées à la revendication 1.

7. Mélange de composants de copulation, contenant les composés des formules 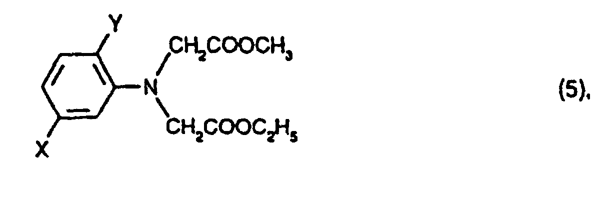 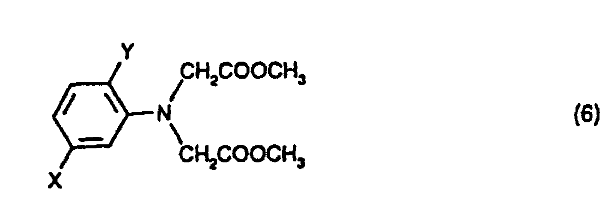 et 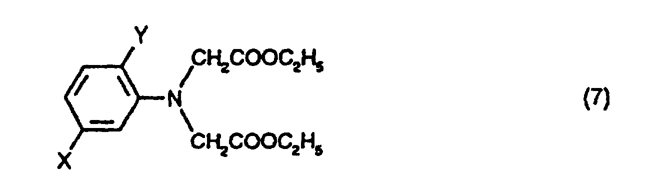 dans lesquelles X représente respectivement un atome d'hydrogène, d'halogène, CF₃, R₃, OR₃, NH-CO-R₇, NH-CO-OR₈, NH-SO₂-R₇ ou NHCO-NR₄R₅, dans lesquels R₃ représente un groupe alkyle en C₁ à C₆, R₄ et R₅ représentent indépendamment l'un de l'autre respectivement un atome d'hydrogène, un groupe alkyle en C₁ à C₄ ou alcoxy en C₁ à C₄-alkyle en C₂ à C₄, R₇ représente un atome d'hydrogène, un groupe alkyle en C₁ à C₆, alcoxy en C₁ à C₄-alkyle en C₁ à C₄ ou phényle et R₈ représente un groupe alkyle en C₁ à C₆ ou alcoxy en C₁ à C₄-alkyle en C₂ à C₄, et
Y représente respectivement un atome d'hydrogène, d'halogène, un groupe méthoxy, éthoxy ou O(CH₂)ₙ-OR₉,
dans lequel R₉ représente un atome d'hydrogène, un groupe méthyle ou CH₂CH₂CN et n est un nombre entier de 1 à 6.

8. Procédé pour la préparation du mélange des composants de copulation selon la revendication 7, **caractérisé en ce qu'**on met à réagir une aniline de formule 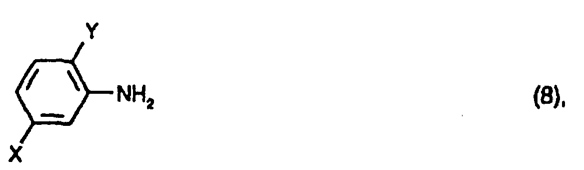 dans laquelle
X et Y présentent les significations indiquées à la revendication 7, avec un mélange de chloroacétate de méthyle et de chloroacétate d'éthyle dans le rapport de 95:5 à 5:95.

9. Utilisation des mélanges de colorants selon la revendication 1 pour la teinture ou l'impression de matériaux en fibres hydrophobes semi-synthétiques ou synthétiques.

10. Utilisation selon la revendication 9, dans laquelle le matériau en fibres hydrophobes semi-synthétiques ou synthétiques est un matériau textile.

11. Utilisation selon la revendication 10 pour la teinture ou l'impression de matériaux textiles de fibres de polyester.

12. Procédé pour la teinture ou l'impression de matériaux en fibres hydrophobes semi-synthétiques ou synthétiques, **caractérisé en ce qu'**on dépose le mélange de colorants selon la revendication 1 sur le matériau mentionné ou qu'on incorpore celui-ci.

13. Procédé selon la revendication 12, dans lequel le matériau en fibres hydrophobes est constitué de fibres de polyester.

14. Matériau teinté ou imprimé selon la revendication 12 ou 13.
